# EUROPÄISCHE PATENTSCHRIFT

(11) **EP 2 984 069 B1**
(45) Veröffentlichungstag und Bekanntmachung des Hinweises auf die Patenterteilung: **11.10.2017**
(21) Anmeldenummer: 14716801.7
(22) Anmeldetag: 09.04.2014
(51) Int. Cl.: C07C 263/10, C07C 209/78, B01D 17/04, C07C 209/68, C07C 209/86, B01D 17/00

(54) **VERFAHREN ZUR HERSTELLUNG VON DI- UND POLYAMINEN DER DIPHENYLMETHANREIHE**
METHOD FOR THE PREPARATION OF DI- AND POLYAMINES OF THE DIPHENYL METHANE SERIES
PROCÉDÉ DESTINÉ À LA FABRICATION DE DI- ET POLYAMINES DE LA SÉRIE DIPHÉNYLMÉTHANE

(30) Priorität: 11.04.2013 EP 13163356
(43) Veröffentlichungstag der Anmeldung: 17.02.2016
(73) Patentinhaber: Covestro Deutschland AG, 51373 Leverkusen (DE)
(72) Erfinder: KNAUF, Thomas, 41542 Dormagen (DE); LORENZ, Wolfgang, 41540 Dormagen (DE); WERSHOFEN, Stefan, 41065 Mönchengladbach (DE); ADAMSON, Richard, 42799 Leichlingen (DE); BECKER, Karsten, 51371 Leverkusen (DE)
(74) Vertreter: Levpat
(86) Internationale Anmeldenummer: PCT/EP2014/057116
(87) Internationale Veröffentlichungsnummer: WO 2014/166977

(56) Entgegenhaltungen:
- EP-A1- 1 616 890
- EP-A1- 2 103 595
- WO-A1-02/053601
- WO-A1-2012/104769
- SULZER: "Liquid-Liquid Separation Technology", Sulzer Chemtech , pages 1-16, Retrieved from the Internet: URL:http://www.sulzer.com/as/-/media/Docum ents/ProductsAndServices/Separation_Techno logy/Coalescers/Brochures/Liquid_Liquid_Se paration_Technology.pdf [retrieved on 2016-11-14]

## Beschreibung

Die Erfindung betrifft ein Verfahren zur Herstellung von Di- und Polyaminen der Diphenylmethanreihe, bei dem zunächst Anilin und Formaldehyd in Abwesenheit eines sauren Katalysators zu einem Reaktionsgemisch enthaltend Aminal und Wasser umgesetzt werden und nach Abtrennung des Wassers das Aminal durch Säurekatalyse zu einem Reaktionsgemisch enthaltend Di- und Polyamine der Diphenylmethanreihe umgesetzt wird, bei dem die Abtrennung des Wassers vom Aminal durch Einsatz eines Koaleszenz-Hilfsmittels unterstützt wird. Erfindungsgemäß wird als Koaleszenz-Hilfsmittel ein Filterbett aus Koaleszenz-Faser-Material eingesetzt.

Die Herstellung von Di- und Polyaminen der Diphenylmethanreihe (MDA) durch Umsetzung von Anilin mit Formaldehyd in Gegenwart saurer Katalysatoren ist allgemein bekannt. Im Sinne der vorliegenden Erfindung werden unter Di- und Polyaminen der Diphenylmethanreihe Amine und Gemische von Aminen folgenden Typs verstanden:

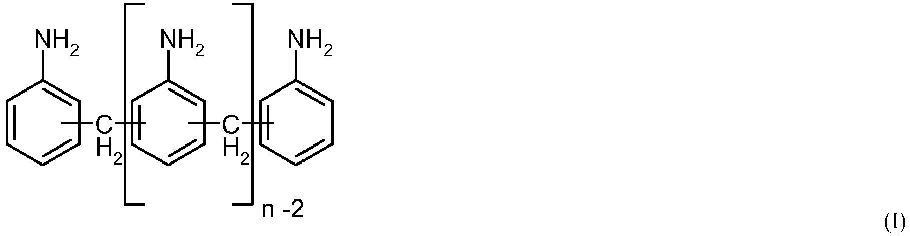

Dabei steht n für eine natürliche Zahl ≥ 2. Im Folgenden werden die Verbindungen dieses Typs, bei denen n = 2 ist, als *Diamine der Diphenylmethanreihe* oder *Diaminodiphenylmethane* (nachfolgend MMDA) bezeichnet. Verbindungen dieses Typs, bei denen n > 2 ist, werden im Rahmen dieser Erfindung als *Polyamine der Diphenylmethanreihe* oder *Polyphenylenpolymethylenpolyamine* (nachfolgend PMDA) bezeichnet. Gemische aus beiden Typen werden als *Di- und Polyamine der Diphenylmethanreihe* bezeichnet (nachfolgend MDA). Die korrespondierenden Isocyanate, die sich formal durch Ersatz aller NH₂-Gruppen durch NCO-Gruppen aus den Verbindungen der Formel (I) ableiten lassen, werden dementsprechend als *Diisocyanate der Diphenylmethanreihe* (nachfolgend MMDI), *Polyisocyanate der Diphenylmethanreihe* oder *Polyphenylenpolymethylenpolyisocyanate* (nachfolgend PMDI) bzw. *Di- und Polyisocyanate der Diphenylmethanreihe* (nachfolgend MDI) bezeichnet. Das Polymere (n > 2) liegt dabei sowohl beim Amin als auch beim Isocyanat in der Regel immer im Gemisch mit dem Dimeren (n = 2) vor, so dass in der Praxis nur zwei Verbindungstypen relevant sind, die reinen Dimeren (MMDA bzw. MMDI) und das Gemisch aus Dimeren und Polymeren (MDA bzw. MDI).

Industriell werden die Di- und Polyamingemische überwiegend durch Phosgenierung zu den entsprechenden Di- und Polyisocyanaten der Diphenylmethanreihe umgesetzt. Die kontinuierliche oder teilweise diskontinuierliche Herstellung von MDA ist z. B. in US-A-5286760, EP-A-451442 und WO-A-99/40059 offenbart.

Die Aufarbeitung des in der Herstellung erhaltenen sauren Reaktionsgemisches wird gemäß dem Stand der Technik durch Neutralisation mit einer Base eingeleitet. Nach dem Stand der Technik erfolgt die Neutralisation üblicherweise bei Temperaturen von beispielsweise 90 °C bis 100 °C ohne Zusatz weiterer Substanzen. (H.J. Twitchett, Chem. Soc. Rev. 3(2), S. 223 (1974)). Sie kann aber auch auf einem anderen Temperaturniveau erfolgen, um z. B. den Abbau von störenden Nebenprodukten zu beschleunigen. Hydroxide der Alkali- und Erdalkalielemente sind als Basen geeignet. Vorzugsweise kommt wässrige NaOH zum Einsatz.

Im Anschluss an die Neutralisation wird in einem Trennbehälter die organische von der wässrigen Phase getrennt. Die nach Abtrennung der wässrigen Phase verbleibende, Roh-MDA enthaltende organische Phase wird weiteren Aufarbeitungsschritten unterzogen, wie z. B. einer Wäsche mit Wasser (Basenwäsche), um Restsalze aus dem Roh-MDA zu waschen. Abschließend wird das so gereinigte Roh-MDA von überschüssigem Anilin, Wasser und anderen im Gemisch vorhandenen Stoffen (z. B. weiteren Lösungsmitteln) durch geeignete Verfahren wie z. B. Destillation, Extraktion oder Kristallisation befreit. Die nach dem Stand der Technik übliche Aufarbeitung ist beispielsweise offenbart in EP 1652 835 A1, Seite 3, Zeile 58 bis Seite 4, Zeile 13 und EP 2 103 595 A1, Seite 7, Zeile 21 bis 37.

EP 1 616 890 A1 offenbart ein Verfahren, in dem Anilin und Formaldehyd zunächst in Abwesenheit des sauren Katalysators zu Aminal umgesetzt werden, und das so erhaltene Aminal anschließend mit saurem Katalysator versetzt und bei Temperaturen von 20 °C bis 100 °C und bei Wassergehalten des so erhaltenen sauren Reaktionsgemisches von 0 bis 20 Gewichtsprozent weiter umgesetzt wird. Insbesondere wird nach der Kondensation von Formaldehyd und Anilin zum Aminal zunächst das Wasser zumindest teilweise aus dem Aminal entfernt, wobei man einen Wassergehalt von 0 bis 5 Gewichtsprozent im Aminal einstellt, bevor das Aminal mit saurem Katalysator versetzt wird. So kann MDA bei einem Protonierungsgrad von < 15 %, bevorzugt 4 % bis 14 %, besonders bevorzugt 5 % bis 13 %, hergestellt werden. Als Protonierungsgrad wird dabei bei einprotonigen sauren Katalysatoren (wie Salzsäure) das molare Verhältnis aus der Menge an eingesetztem sauren Katalysator und der im Reaktionsgemisch vorhandenen molaren Menge an Aminfunktionen bezeichnet.

EP 1 813 598 A1 lehrt (siehe insbesondere die Absätze [0037] bis [0042]), dass das in der Aminalreaktion entstandene Reaktionswasser teilweise abgetrennt und mit anderen Abwasserströmen des Prozesses vereinigt und zur Entfernung von organischen Bestandteilen, wie z. B. Anilin und MDA, weiter behandelt wird, wie z. B. einer Kombination aus Extraktion und Destillation. Der Verbleib des Einsatzstoffes Formalin wird nicht beschrieben.

Die Güte eines Verfahrens zur Herstellung von MDA ist einerseits definiert durch den Gehalt des Produktes an unerwünschten Nebenprodukten der Reaktion. Andererseits ist die Güte eines kontinuierlichen Verfahrens dadurch definiert, dass der gesamte Prozess von Anfahren, normaler Produktion bis zum Abfahren des Prozesses ohne technischen Produktionsausfall oder Problemen, die zu einem Eingriff in den Prozess nötigen, betrieben werden kann, und dass es nicht zu Verlusten an Einsatzstoffen, Zwischenprodukten oder an Endprodukt kommt. Solche Probleme können z. B. bei der Einstellung des Wassergehaltes im Aminal durch Phasentrennung von organischer und wässriger Phase auftreten. Derartige Probleme können z. B. sein, dass es zu Verzögerungen bei der Phasentrennung kommt, oder dass die Phasentrennung unvollständig ist, oder dass sich eine dritte Phase (Mulm bzw. Mulm*schicht*) ausbildet. Diese dritte Phase ist eine stabile, ggf. voluminöse Zwischenphase, die zwischen der wässrigen und der organischen Phase auftritt und die Phasentrennung erschwert und im Extremfall sogar vollständig verhindert. Im für den betrieblichen Ablauf ungünstigsten Fall müssen der oder die betroffenen Phasentrennbehälter vollständig entleert und gereinigt werden. Der Inhalt des bzw. der Phasentrennbehälter ist dann aufwändig aufzuarbeiten oder zu entsorgen, was mit erheblichen Kosten verbunden ist. Dies kann unter Umständen auch dazu führen, dass die kontinuierliche Produktion unterbrochen werden muss. Wenn sich die Bildung einer Mulmschicht nicht völlig vermeiden lässt, so wird diese schließlich in eine der beiden Phasen gelangen. Gelangt die Mulmschicht in die organische Phase, so ist dies im Fall der Phasentrennung nach der Aminalreaktion weniger bedenklich als wenn sie in die wässrige Phase gelangt. Denn im letztgenannten Fall gelangen dann mit der Mulmschicht größere Mengen dispers gelöster Organika in das Aminalwasser. Besagte Verluste können dann bei der Entsorgung oder Weiterverwendung des Aminalwassers auftreten. Es wäre daher wünschenswert, verfahrenstechnische Maßnahmen zur Verfügung zu haben, um diese Problematik beherrschen zu können.

EP 2 103 595 A1 befasst sich mit einer Verfahrensführung zur Herstellung von Di- und Polyaminen der Diphenylmethanreihe, bei welcher Anilin und Formaldehyd in Gegenwart eines sauren Katalysators umgesetzt werden. Im Zusammenhang mit der Phasentrennung nach der Neutralisation des Rohprodukts wird offenbart, dass diese Phasentrennung durch Zugabe von Wasser und/oder Anilin unterstützt werden kann. Bevorzugt wird das durch Zugabe von Wasser und/oder Anilin verdünnte Reaktionsgemisch in Scheideflaschen mit die Koaleszenz der beiden Phasen unterstützenden Plattenpaketen als Einbauten in eine organische und wässrige Phase getrennt (Absätze [0043] und [0044]). Wenn besonders hohe Anforderungen an die Güte einer Phasentrennung gestellt werden, lassen sich mit Plattenpaketen keine vollständig zufriedenstellenden Ergebnisse erzielen. Dies wurde in EP 2 103 595 A1 nicht erkannt.

Den beschriebenen Verfahren des Standes der Technik gelingt es zwar, mit hoher Ausbeute MDA herzustellen, jedoch werden keine technischen Hilfsmittel beschrieben, die die Abtrennung der organischen von der wässrigen Aminalphase mit der wünschenswerten Wirksamkeit verbessern könnten, um die Verluste an Einsatzstoffen und Zwischenprodukten in dem Reaktionsprozess zu minimieren und einen reibungslosen technischen Ablauf des Produktionsprozesses zu gewährleisten.

WO 02/053601 A1 befasst sich mit einem Verfahren zur Herstellung hochreaktiver Polyisobutene. In diesem Zusammenhang wird der Einsatz von Koaleszens-fördernden Einbauten bei der Trennung von wässrigen und organischen Phasen offenbart.

Der Prospekt *Sulzer Chemtech "Liquid-Liquid Separation Technology"* der Fa. Sulzer stellt verschiedene Trennhilfen vor. Ob und unter welchen Bedingungen solche Trennhilfen in der MDA-Herstellung eingesetzt werden können, ist dem Prospekt nicht zu entnehmen.

Es bestand also ein Bedarf an einem Verfahren zur Herstellung von Di- und Polyaminen der Diphenylmethanreihe, bei dem es durch einfache Maßnahmen möglich ist, in der Aminalstufe eine verbesserte Phasentrennung zwischen organischer Phase und wässriger Phase herbeizuführen. Dies würde die Wirtschaftlichkeit bestehender MDA-Prozesse verbessern.

Dem vorstehend Gesagten Rechnung tragend ist ein Gegenstand der vorliegenden Erfindung ein Verfahren zur Herstellung von Di- und Polyaminen der Diphenylmethanreihe, bei dem
a) Anilin und Formaldehyd in Abwesenheit eines sauren Katalysators zu einem Reaktionsgemisch enthaltend Aminal und Wasser umgesetzt werden,
b) Wasser, das im Wesentlichen aus Kondensationswasser der Aminalreaktion und Wasser aus dem Einsatzstoff Formaldehyd besteht, zumindest teilweise aus dem in Schritt a) erhaltenen Reaktionsgemisch abgetrennt wird, wobei eine organische, das Aminal enthaltende Phase (**1**) erhalten wird,
c) die in Schritt b) erhaltene organische, das Aminal enthaltende Phase (**1**) in Gegenwart eines sauren Katalysators umgesetzt wird, wobei ein Reaktionsgemisch enthaltend Di- und Polyamine der Diphenylmethanreihe erhalten wird,
d) das in Schritt c) erhaltene Reaktionsgemisch enthaltend Di- und Polyamine der Diphenylmethanreihe neutralisiert und anschließend einer Aufarbeitung umfassend Wäsche und Destillation unterzogen wird,
wobei zur Abtrennung des Wassers aus dem in Schritt a) erhaltenen Reaktionsgemisch
b.1) das in Schritt a) erhaltene Reaktionsgemisch in einem Trennbehälter in eine wässrige und eine organische Phase (**1a**) getrennt wird, wobei in der wässrigen Phase organische Bestandteile dispergiert sind, und dann
b.2) die in Schritt b.1) erhaltene wässrige Phase (das sog. "Aminalwasser")
   (i) bei einer Temperatur im Bereich von 50 °C bis 120 °C und bei einem solchen Druck, dass ein Sieden des dispersen Systems unterbleibt, ohne Verwendung von Inertgasen durch ein Filterbett aus Koaleszenz-Faser-Material geführt und
   (ii) anschließend in eine wässrige Phase und eine organische Phase (**1b**) getrennt wird, und dann
b.3) die in Schritt b.2) erhaltene organische Phase (**1b**) mit der in Schritt b.1) erhaltenen organischen Phase (**1a**) zur organischen Phase (**1**) vereint wird.

Die Erfindung betrifft auch ein Verfahren zur Herstellung von Di- und Polyisocyanaten der Diphenylmethanreihe, bei dem Di- und Polyamine der Diphenylmethanreihe nach dem erfindungsgemäßen Verfahren hergestellt werden und anschließend mit Phosgen zu den entsprechenden Di- und Polyisocyanaten umgesetzt werden.

Nachstehend werden Ausführungsformen der Erfindung näher beschrieben. Dabei können verschiedene Ausführungsformen beliebig miteinander kombiniert werden, sofern sich für den Fachmann aus dem Kontext nicht eindeutig das Gegenteil ergibt.

Die Kondensation von Anilin und Formaldehyd in **Schritt a)** kann nach einem Verfahren nach dem Stand der Technik durchgeführt werden. Dabei werden bevorzugt Anilin und wässrige Formaldehydlösung bei einem molaren Verhältnis Anilin zu CH₂O von 1,7 : 1 bis 20 : 1, bevorzugt 1,7 : 1 bis 5,0 : 1 bei einer Temperatur von 20 °C bis 100 °C, bevorzugt von 30 °C bis 95 °C, besonders bevorzugt von 40 °C bis 90 °C zu Aminal und Wasser kondensiert. Die Umsetzung erfolgt üblicherweise bei Normaldruck. Geeignete Anilinqualitäten sind z. B. beschrieben in EP 1 257 522 B1, EP 2 103 595 A1 und EP 1 813 598 B1. Es werden bevorzugt technische Qualitäten an Formalin (wässrige Lösung von Formaldehyd) mit 30 Massen-% bis 50 Massen-% Formaldehyd in Wasser eingesetzt. Jedoch sind auch Formaldehydlösungen mit niedrigeren oder höheren Konzentrationen oder auch der Einsatz gasförmigen Formaldehyds denkbar.

In **Schritt b)** erfolgt die Phasentrennung von organischer Aminalphase (**1.a**) und wässriger Phase (**Schritt b.1)**) bei einer Temperatur von 20 °C bis 100 °C, bevorzugt von 30 °C bis 95 °C, besonders bevorzugt von 40 °C bis 90 °C, bevorzugt bei Umgebungsdruck. Die Optimierung dieser Phasentrennung erfolgt durch den Einsatz eines Filterbetts aus Koaleszenz-Faser-Material als Koaleszens-Hilfsmittel in **Schritt b.2).** Hierbei wird der organische Anteil in dem nach der Aminalreaktion separierten Aminalwasser minimiert (Klärung der Resttrübe). Der so separierte organische Anteil (**1**.**b**) wird mit der in **Schritt b.1)** erhaltenen organischen Phase (**1.a**) zur organischen Phase (**1**) vereint.

Die Abtrennung der organischen Bestandteile (**1**.**b**) aus der wässrigen Phase (**Aminalwasser**) erfolgt erfindungsgemäß durch Einsatz eines Filterbetts aus Koaleszenz-Faser-Material als Koaleszenz-Hilfsmittel. Die Wahl des Faser-Materials ist unter anderem abhängig von:
- Benetzungseigenschaften der dispersen Phase (Tropfen) auf dem Fasermaterial,
- der Grenzflächenspannung des Stoffsystems,
- der Viskosität beider Phasen des Stoffsystems.

Die feindispersen organischen Tröpfchen müssen die Oberfläche des Fasermaterials benetzten können.

Beim Durchtritt der Flüssig-flüssig-Dispersion (organische Bestandteile dispergiert im Aminalwasser) durch das Fasermaterial vermögen die feindispers vorliegenden organischen Tröpfchen die Faseroberfläche zu benetzen. Die organischen Tröpfchen sammeln sich auf den Fasern an (Tropfen-Faser-Koaleszenz), nach weiterer Faserbelegung verringern sich die Abstände zwischen den angelagerten kleinen Tröpfchen, und schließlich vereinigen sich die Tröpfchen zu größeren Tropfen (Tropfen-Tropfen-Koaleszenz). Bei Überschreiten eines charakteristischen Grenztropfendurchmessers (abhängig vom Stoffsystem, Viskosität, Strömungsbedingungen) werden die nun vergrößerten Tropfen durch die Strömungskräfte im Faserbett abgelöst und treten als deutlich vergrößerte Tropfen im Vergleich zum Eintrittstropfen aus dem Fasermaterial heraus. Aufgrund der verbesserten Sedimentationseigenschaften können diese organischen Tropfen in der sich anschließenden Phasentrennung im Erdschwerefeld abgeschieden werden, was zu einer Minimierung der Resttrübe des Aminalwassers führt.

Der Erfolg bei der Trennaufgabe hängt davon ab, dass eine Bildung von Gasblasen vermieden wird, was eine Prozessführung bei Temperaturen unter dem Siedepunkt des dispersen Systems und der einzelnen resultierenden Phasen erfordert und die Verwendung von Inertgasen ausschließt. Daher wird die Trennaufgabe für das erfindungsgemäße System (**Schritt (b.2 (i)**) im Temperaturbereich von 50 °C bis 120 °C, bevorzugt von 70 °C bis 115 °C und besonders bevorzugt von 75 °C bis 110 °C durchgeführt. Für die Trennaufgabe wird der Druck im Trennsystem so gewählt, dass ein Sieden des dispersen Systems unterbleibt. Der einzustellende Mindestdruck hängt vom Temperaturniveau und der Zusammensetzung des dispersen Systems ab und kann durch einfache Versuche ermittelt werden. Bevorzugt wird die Trennaufgabe bei Atmosphärendruck bis zu einem erhöhten Druck von 10 bar absolut, bevorzugt bis zu 5 bar absolut, besonders bevorzugt bis zu 2 bar absolut, durchgeführt.

Der Faserdurchmesser des Koaleszenz-Faser-Materials beträgt bevorzugt von 1,0 µm bis 150 µm, besonders bevorzugt von 1,0 µm bis 100 µm, ganz besonders bevorzugt von 2,0 µm bis 30 µm. Die nominelle Porengröße des Koaleszenzmaterials liegt bevorzugt bei 5 µm bis 40 µm und besonders bevorzugt bei 10 µm bis 30 µm.

Für die Abtrennung der dispers vorliegenden, organischen Tröpfchen werden bevorzugt Fasern aus einem Borosilikat-Glasmaterial oder organischem Polymermaterial, besonders bevorzugt Fasern aus einem organischen Polymermaterial, das im alkalischen Medium beständig ist, eingesetzt. Geeignete organische Polymermaterialien sind bspw. Polytetrafluorethylen (PTFE), Polytrifluorethylen, Polydifluorethylen, Copolymerisate von Tetrafluorethylen und /oder Trifluorethylen und/oder Difluorethylen untereinander und mit anderen Monomeren, Derivate des PTFE, Polypropylen (PP) und Polyethylen (PE). Erfindungsgemäß werden unter "Derivaten des PTFE" solche Polymermaterialien verstanden, die PTFE enthalten (z. B. Komposite mit PTFE als einem der Bestandteile) oder Copolymerisate des Tetrafluorethylens mit anderen Monomeren (z. B. Poly(ethylen-co-tetrafluorethylen) (ETFE)) sind. Insbesondere die fluorhaltigen Polymermaterialien eignen sich hervorragend für den erfindungsgemäßen Zweck, und zwar bei allen in der Praxis relevanten Temperaturen. Ab einer Temperatur von ca. 80 °C sind die fluorhaltigen Polymermaterialien gegenüber anderen Polymermaterialien wie PP oder PE bevorzugt. Ganz besonders bevorzugt wird ein Material aus Polytetrafluorethylen (PTFE) oder dessen Derivaten (insbesondere ETFE und Komposite enthaltend PTFE oder ETFE) eingesetzt. Die spezifische hydraulische Belastung beim Durchführen des Aminalwassers durch das Koaleszenz-Fasermaterial liegt bevorzugt im Bereich 1,0 m³/(m²h) bis 10 m³/(m²h), besonders bevorzugt 1,0 m³/(m²h) bis 8,0 m³/(m²h) und ganz besonders bevorzugt 2,0 m³/(m²h) bis 6,0 m³/(m²h).

Die Stärke des erfindungsgemäßen Filterbettes aus Koaleszenz-Fasermaterial beträgt bevorzugt 1,0 mm bis 100 mm, besonders bevorzugt 1,0 mm bis 50 mm und ganz besonders bevorzugt 1,0 mm bis 30 mm.

Die Umlagerung des Aminals in **Schritt c)** erfolgt in Gegenwart eines sauren Katalysators, üblicherweise einer starken Mineralsäure wie Salzsäure. Bevorzugt ist der Einsatz von Mineralsäure in einem molaren Verhältnis Mineralsäure: Anilin von 0,001 : 1 bis 0,9 : 1, bevorzugt 0,05 : 1 bis 0,5 : 1. Es können natürlich auch feste, saure Katalysatoren, wie in der Literatur beschrieben, verwendet werden. Dabei kann Formaldehyd in eine Mischung von Anilin und saurem Katalysator gegeben und die Reaktionslösung durch stufenweises Erhitzen ausreagiert werden. Alternativ können auch Anilin und Formaldehyd zunächst vorreagiert werden und anschließend mit oder ohne vorhergehender Wasserabtrennung mit dem sauren Katalysator oder einem Gemisch von weiterem Anilin und saurem Katalysator versetzt werden, wonach die Reaktionslösung durch stufenweises Erhitzen ausreagiert wird. Diese Reaktion kann kontinuierlich oder diskontinuierlich nach einem der zahlreichen in der Literatur beschriebenen Verfahren ausgeführt werden (z. B. in EP 1 616 890 A1 oder EP 127 0544 A1).

In **Schritt d)** wird das Reaktionsgemisch enthaltend die Di- und Polyamine der Diphenylmethanreihe, ggf. unter Zusatz von Wasser und / oder Anilin, zunächst neutralisiert (**Schritt d.1)**). Die Neutralisation erfolgt bevorzugt bei einer Temperatur von 90 °C bis 100 °C ohne Zusatz weiterer Substanzen. Sie kann aber auch auf einem anderen Temperaturniveau erfolgen, um z. B. den Abbau von störenden Nebenprodukten zu beschleunigen. Als Basen eignen sich bevorzugt die Hydroxide der Alkali- und Erdalkalielemente. Vorzugsweise kommt Natronlauge zur Anwendung. Die zur Neutralisation eingesetzte Base wird bevorzugt in einer Menge von mehr als 100 %, besonders bevorzugt 105 % bis 120 %, der für die Neutralisation des eingesetzten sauren Katalysators stöchiometrisch benötigten Menge eingesetzt (siehe EP 1 652 835 A1). Das so erhaltene zweiphasige Gemisch wird nun in eine organische Phase enthaltend Di- und Polyamine der Diphenylmethanreihe und eine wässrige Phase aufgetrennt. Dies kann durch die Zugabe von Anilin und / oder Wasser unterstützt werden. Wird die Phasentrennung durch Zugabe von Anilin und / oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und ggf. durch Zugabe von Anilin und / oder Wasser verdünnte Reaktionsgemisch wird anschließend bevorzugt einem Apparat zugeführt, der aufgrund seiner Konfiguration und / oder Einbauten besonders zur Auftrennung in eine organische Phase enthaltend MDA und eine wässrige Phase geeignet ist, bevorzugt Phasentrenn-oder Extraktionsvorrichtungen entsprechend dem Stand der Technik, wie sie beispielsweise in Mass-Transfer Operations, 3rd Edition, 1980, McGraw-Hill Book Co, S. 477 bis 541, oder Ullmann's Encyclopedia of Industrial Chemistry (Vol. 21, Liquid-Liquid Extraction, E. Müller et al., Seite 272-274, 2012 Wiley-VCH Verlag GmbH & Co. KGaA, Weinheim, DOI: 10.1002/14356007.b03_06.pub2) oder in Kirk-Othmer Encyclopedia of Chemical Technology (siehe "http://onlinelibrary.wiley.com/book/10.1002/0471238961", Published Online: 15 Juni 2007, Seite 22-23) beschrieben sind (Mixer-Settler-Kaskade oder Absetzbehälter).

Die so erhaltene organische Phase wird nun einer Wäsche unterzogen (**Schritt d**.**2)**). Als Waschflüssigkeit wird bevorzugt Wasser eingesetzt. Das Waschwasser wird im Anschluss mittels Phasentrennung abgeschieden. Auf diese Weise wird der Salzgehalt der organischen Phase erniedrigt. Ein geeignetes Verfahren ist bspw. in DE-A-2549890, Seite 3, beschrieben. Die in **Schritt d.2)** erhaltene organische Phase hat bevorzugt eine Zusammensetzung, bezogen auf die Gesamtmasse dieser organischen Phase, von 5,0 Massen-% bis 15 Massen-% Wasser, und, je nach Einsatzverhältnissen von Anilin und Formaldehyd, 5,0 Massen-% bis 90 Massen-%, bevorzugt 5,0 Massen-% bis 40 Massen-%, Anilin und 5,0 Massen-% bis 90 Massen-%, bevorzugt 50 Massen-% bis 90 Massen-%, Di- und Polyamine der Diphenylmethanreihe. Nach Austritt aus der Phasentrennung in **Schritt d.2)** hat die organische Phase enthaltend Di- und Polyamine der Diphenylmethanreihe üblicherweise eine Temperatur von 80 °C bis 150 °C.

Aus der so erhaltenen, neutralisierten und gewaschenen, organischen Phase enthaltend Di- und Polyamine der Diphenylmethanreihe wird nun wie im Stand der Technik bekannt destillativ Wasser und Anilin abgetrennt (**Schritt d**.**3)**). Dies geschieht bevorzugt wie in EP 1 813 597 B1, insbesondere in den Absätzen [0014] bis [0043], beschrieben.

Die so erhaltenen Di- und Polyamine der Diphenylmethanreihe können nach den bekannten Methoden mit Phosgen zu den entsprechenden Di- und Polyisocyanaten der Diphenylmethanreihe umgesetzt werden. Dabei kann die Phosgenierung nach einem der aus dem Stand der Technik bekannten Verfahren durchgeführt werden (z. B. DE-A-844 896 oder DE-A-198 17 691).

Wenn der Organikagehalt des Aminalwassers mit Hilfe eines Filterbetts aus Koaleszenz-Faser-Material reduziert, und die so gewonnen Organika erfindungsgemäß weiterverarbeitet werden, ergeben sich unter anderem folgende Vorteile:
i) Die Herstellkosten des Verfahrens werden verbessert, weil die Verluste an Einsatzstoffen und Zwischenprodukten über die wässrige Phase minimiert werden.
ii) Die reduzierte Belastung der wässrigen Phase mit Organika führt zu einem geringeren Behandlungsaufwand in der Abwasseraufbereitung (Energiekosten werden eingespart, weil weniger Dampf für das Abstrippen von Organika aus dem MDA-Abwasser benötigt wird).

### Beispiele:

### Beispiel (erfindungsgemäß)

In einem kontinuierlichen Reaktionsprozess (**Schritt a)**) wurden 24,4 t/h "Einsatzanilin" (90 Massen-% Anilin) und 6,1 t/h 50 %ige Formaldehydlösung, die 1,0 Massen-% Methanol enthielt, vermischt und bei 95 °C in einem gerührten Reaktionskessel kontinuierlich zum Aminal umgesetzt. Die anschließende Phasentrennung (**Schritt b.1)**) in einem Phasentrennapparat gestaltete sich schwierig, da die Phasentrennschicht wegen einer Trübung in der wässrigen Phase schlecht erkennbar war. Die untere, wässrige Phase wurde anschließend in **Schritt b.2)** durch ein Koaleszenz-Hilfsmittel geführt. Als Koaleszenz-Hilfe wurde ein Koaleszenz-Fasermaterial aus einem in alkalischem Medium beständigen organischen Polymermaterial (PTFE) eingesetzt. Der Faserdurchmesser des Materiales betrug 20 µm. Es wurden 11 übereinanderliegende, durchströmte Faserfliese benutzt. Die Durchflussgeschwindigkeit betrug 4 m³/(m²h), d. h., 4 m³/h Durchsatz bezogen auf eine Durchströmquerschnittsfläche von 1 m². Die geklärte wässrige Phase war nach Durchtritt des Fasermaterials klar. Die Resttrübeklärung mittels Koaleszenz-Fasermaterial konnte stabil über eine lange Produktionsphase betrieben werden.

Die organischen Phasen aus **Schritt b.1)**, der Hauptphasentrennung, und aus **Schritt b.2)**, der Resttrübeklärung, wurden vereinigt (**Schritt b**.**3)**).

Nach der Phasentrennung zur Entfernung der wässrigen Phase wurden die vereinigten organischen Phasen mit 31 %iger wässriger Salzsäure versetzt (Protonierungsgrad 10 %, d. h., pro mol Aminogruppen wurden 0,1 mol HCl zugegeben) und bei 50 °C bis 150 °C in einer Reaktorkaskade umgesetzt (**Schritt c)**). Nach vollständiger Reaktion wurde das erhaltene Reaktionsgemisch mit 32 %iger Natronlauge im molaren Verhältnis von 1,1 : 1 Natronlauge zu HCl versetzt und in einem Neutralisationsrührbehälter umgesetzt (**Schritt d)**). Die Temperatur betrug 115 °C. Der absolute Druck betrug 1,4 bar. Die neutralisierte Basenmischung wurde anschließend in einem Neutralisationsabscheider in eine wässrige, untere Phase, die zu einem Abwassersammelbehälter geführt wurde, und in eine organische Phase getrennt. Die organische, obere Phase, wurde zur Wäsche geleitet. In einem gerührten Waschbehälter wurde das alkalische MDA mit Kondensat gewaschen. Nach Abtrennung des Waschwassers in einem Waschwasserabscheider wurde das so erhaltene Roh-MDA durch Destillation von Wasser und Anilin befreit, wobei als Sumpfprodukt 17 t/h MDA erhalten wurden.

## Patentansprüche

1. Verfahren zur Herstellung von Di- und Polyaminen der Diphenylmethanreihe, bei dem
a) Anilin und Formaldehyd in Abwesenheit eines sauren Katalysators zu einem Reaktionsgemisch enthaltend Aminal und Wasser umgesetzt werden,
b) Wasser zumindest teilweise aus dem in Schritt a) erhaltenen Reaktionsgemisch abgetrennt wird, wobei eine organische, das Aminal enthaltende Phase (**1**) erhalten wird,
c) die in Schritt b) erhaltene organische, das Aminal enthaltende Phase (**1**) in Gegenwart eines sauren Katalysators umgesetzt wird, wobei ein Reaktionsgemisch enthaltend Di- und Polyamine der Diphenylmethanreihe erhalten wird,
d) das in Schritt c) erhaltene Reaktionsgemisch enthaltend Di- und Polyamine der Diphenylmethanreihe neutralisiert und anschließend einer Aufarbeitung umfassend Wäsche und Destillation unterzogen wird,
**dadurch gekennzeichnet, dass**
zur Abtrennung des Wassers aus dem in Schritt a) erhaltenen Reaktionsgemisch
b.1) das in Schritt a) erhaltene Reaktionsgemisch in einem Trennbehälter in eine wässrige und eine organische Phase (**1a**) getrennt wird, wobei in der wässrigen Phase organische Bestandteile dispergiert sind, und dann
b.2) die in Schritt b.1) erhaltene wässrige Phase
(i) bei einer Temperatur im Bereich von 50 °C bis 120 °C und bei einem solchen Druck, dass ein Sieden des dispersen Systems unterbleibt, ohne Verwendung von Inertgasen durch ein Filterbett aus Koaleszenz-Faser-Material geführt und
(ii) anschließend in eine wässrige Phase und eine organische Phase (**1b**) getrennt wird, und dann
b.3) die in Schritt b.2) erhaltene organische Phase (**1b**) mit der in Schritt b.1) erhaltenen organischen Phase (**1a**) zur organischen Phase (**1**) vereint wird.

2. Verfahren nach Anspruch 1, bei dem die Fasern des Koaleszenz-Faser-Materials aus einem Material ausgewählt aus der Gruppe bestehend aus Glas und organischem Polymermaterial gefertigt sind.

3. Verfahren nach Anspruch 2, bei dem das Koaleszenz-Faser-Material ausgewählt ist aus der Gruppe bestehend aus Borosilikatglas und fluorhaltigen organischen Polymermaterialien.

4. Verfahren nach Anspruch 3, bei dem das fluorhaltige organische Polymermaterial ausgewählt ist aus der Gruppe bestehend aus Polytetrafluorethylen (PTFE), Polytrifluorethylen, Polydifluorethylen, Copolymerisaten von Tetrafluorethylen und /oder Trifluorethylen und/oder Difluorethylen untereinander und mit anderen Monomeren, und Derivaten des Polytetrafluorethylens.

5. Verfahren nach Anspruch 4, bei dem das fluorhaltige organische Polymermaterial ausgewählt ist aus der Gruppe bestehend aus Polytetrafluorethylen und Derivaten des Polytetrafluorethylens.

6. Verfahren nach einem der Ansprüche 1 bis 5, bei dem die Stärke des Filterbettes aus Koaleszenz-Fasermaterial 1,0 mm bis 100 mm beträgt.

7. Verfahren zur Herstellung von Di- und Polyisocyanaten der Diphenylmethanreihe umfassend die Herstellung von Di- und Polyaminen der Diphenylmethanreihe gemäß einem Verfahren nach einem der Ansprüche 1 bis 6 und Phosgenierung der so hergestellten Di- und Polyamine der Diphenylmethanreihe.

## Claims

1. Process for the preparation of di- and polyamines of the diphenylmethane series, in which
a) aniline and formaldehyde are converted in the absence of an acidic catalyst to a reaction mixture comprising aminal and water,
b) water is removed at least in part from the reaction mixture obtained in step a), giving an organic phase (**1**) comprising the aminal,
c) the organic aminal-comprising phase (**1**) obtained in step b) is converted in the presence of an acidic catalyst, giving a reaction mixture comprising di- and polyamines of the diphenylmethane series,
d) the reaction mixture comprising di- and polyamines of the diphenylmethane series obtained in step c) is neutralized and then subjected to a work-up involving washing and distillation,
**characterized in that**
for the purposes of the separation of the water from the reaction mixture obtained in step a)
b.1) the reaction mixture obtained in step a) is separated in a separating container into an aqueous and an organic phase (**1a**), wherein organic constituents are dispersed in the aqueous phase, and then
b.2) the aqueous phase obtained in step b.1)
(i) is passed at a temperature in the range from 50°C to 120°C and at a pressure such that boiling of the disperse system does not occur, without use of inert gases through a filter bed of coalescence fiber material and
(ii) is then separated into an aqueous phase and an organic phase (**1b**), and then b.3) the organic phase (**1b**) obtained in step b.2) is combined with the organic phase (**1a**) obtained in step b.1) to give the organic phase (**1**).

2. Process according to Claim 1, in which the fibers of the coalescence fiber material are prepared from a material selected from the group consisting of glass and organic polymer material.

3. Process according to Claim 2, in which the coalescence fiber material is selected from the group consisting of borosilicate glass and fluorine-containing organic polymer materials.

4. Process according to Claim 3, in which the fluorine-containing organic polymer material is selected from the group consisting of polytetrafluoroethylene (PTFE), polytrifluoroethylene, polydifluoroethylene, copolymers of tetrafluoroethylene and/or trifluoroethylene and/or difluoroethylene with one another and with other monomers, and derivatives of polytetrafluroethylene.

5. Process according to Claim 4, in which the fluorine-containing organic polymer material is selected from the group consisting of polytetrafluoroethylene and derivatives of polytetrafluoroethylene.

6. Process according to any one of Claims 1 to 5, in which the thickness of the filter bed of coalescence fiber material is 1.0 mm to 100 mm.

7. Process for the preparation of di- and polyisocyanates of the diphenylmethane series comprising the preparation of di- and polyamines of the diphenylmethane series according to a process according to any of Claims 1 to 6 and phosgenation of the di- and polyamines of the diphenylmethane series thus prepared.

## Revendications

1. Procédé pour la préparation de diamines et de polyamines de la série des diphénylméthanes, dans lequel
a) de l'aniline et du formaldéhyde sont transformés en l'absence d'un catalyseur acide en un mélange réactionnel contenant de l'aminal et de l'eau,
b) l'eau est au moins partiellement éliminée du mélange réactionnel obtenu dans l'étape a), une phase (1) organique, contenant l'aminal, étant obtenue,
c) la phase (1) organique, contenant l'aminal, obtenue dans l'étape b) est transformée en présence d'un catalyseur acide, un mélange réactionnel contenant des diamines et des polyamines de la série des diphénylméthanes étant obtenu,
d) le mélange réactionnel obtenu dans l'étape c) contenant des diamines et des polyamines de la série des diphénylméthanes est neutralisé et ensuite soumis à un traitement comprenant un lavage et une distillation,
**caractérisé en ce que** pour la séparation de l'eau du mélange réactionnel obtenu dans l'étape a) b.1)
le mélange réactionnel obtenu dans l'étape a) est séparé dans un récipient de séparation en une phase aqueuse et en une phase organique (1a), des constituants organiques étant dispersés dans la phase aqueuse, et ensuite
b.2) la phase aqueuse obtenue dans l'étape b.1)
(i) est guidée à une température dans la plage de 50°C à 120°C et à une pression telle qu'une ébullition du système dispersé ne se produit pas, sans utiliser de gaz inertes, à travers un lit de filtration constitué par un matériau fibreux de coalescence et
(ii) est ensuite séparée en une phase aqueuse et en une phase organique (1b) et ensuite
b.3) la phase organique (1b) obtenue dans l'étape b.2) est rassemblée avec la phase organique (1a) obtenue dans l'étape b.1) en phase organique (1).

2. Procédé selon la revendication 1, dans lequel les fibres du matériau fibreux de coalescence sont fabriquées à partir d'un matériau choisi dans le groupe constitué par le verre et un matériau polymère organique.

3. Procédé selon la revendication 2, dans lequel le matériau fibreux de coalescence est choisi dans le groupe constitué par le verre borosilicaté et les matériaux polymères organiques contenant du fluor.

4. Procédé selon la revendication 3, dans lequel le matériau polymère organique contenant du fluor est choisi dans le groupe constitué par le polytétrafluoroéthylène (PTFE), le polytrifluoroéthylène, le polydifluoroéthylène, les copolymères de tétrafluoroéthylène et/ou de trifluoroéthylène et/ou de difluoroéthylène les uns avec les autres et avec d'autres monomères et les dérivés du polytétrafluoroéthylène.

5. Procédé selon la revendication 4, dans lequel le matériau polymère organique contenant du fluor est choisi dans le groupe constitué par le polytétrafluoroéthylène et les dérivés du polytétrafluoroéthylène.

6. Procédé selon l'une quelconque des revendications 1 à 5, dans lequel l'épaisseur du lit de filtration en matériau fibreux de coalescence est de 1,0 mm à 100 mm.

7. Procédé pour la préparation de diisocyanates et de polyisocyanates de la série des diphénylméthanes comprenant la préparation de diamines et de polyamines de la série des diphénylméthanes selon un procédé selon l'une quelconque des revendications 1 à 6 et la phosgénation des diamines et des polyamines de la série des diphénylméthanes ainsi préparées.
